Europäisches Patentamt

European Patent Office

Office européen des brevets

(19)

(11) Veröffentlichungsnummer: **0 053 249**
**B1**

(12) **EUROPÄISCHE PATENTSCHRIFT**

(45) Veröffentlichungstag der Patentschrift:
19.10.83

(21) Anmeldenummer: 81108204.9

(22) Anmeldetag: 12.10.81

(51) Int. Cl.³: **C 07 C 51/46**, C 07 C 53/08,
C 07 C 53/12, C 07 C 67/54,
C 07 C 69/14, C 07 C 69/15,
C 07 C 69/16, C 07 C 27/30,
C 07 C 51/573

(54) Verfahren zur Abtrennung organischer Jod-Verbindungen von acetaldehydfreien Carbonylierungsprodukten des Methanols, Methylacetates und Dimethylethers.

(30) Priorität: 29.11.80 DE 3045081

(43) Veröffentlichungstag der Anmeldung:
09.06.82 Patentblatt 82/23

(45) Bekanntmachung des Hinweises auf die Patenterteilung:
19.10.83 Patentblatt 83/42

(84) Benannte Vertragsstaaten:
BE DE FR GB IT NL

(56) Entgegenhaltungen:
DE-A-2 635 935
FR-A-1 490 688
GB-A-980 196

(73) Patentinhaber: BASF Aktiengesellschaft,
Carl-Bosch-Strasse 38, D-6700 Ludwigshafen (DE)

(72) Erfinder: Hartmann, Horst, Lindenstrasse 45,
D-6737 Boehl-Iggelheim (DE)
Erfinder: Hochstein, Waldhelm, Dr., Am Wurmberg 4,
D-6713 Freinsheim (DE)
Erfinder: Kaibel, Gerd, Robert-Bosch-Strasse 4,
D-6840 Lampertheim (DE)
Erfinder: Mueller, Franz-Josef, Dr.,
Mueller-Thurgau-Weg 1, D-6706 Wachenheim (DE)

BUNDESDRUCKEREI BERLIN

**0 053 249**

## Verfahren zur Abtrennung organischer Jod-Verbindungen von acetaldehydfreien Carbonylierungsprodukten des Methanols, Methylacetats und Dimethylethers

Die vorliegende Erfindung betrifft ein neues Verfahren zur Abtrennung organischer Jod-Verbindungen, wie vor allem Methyljodid, von acetaldehydfreien Carbonylierungsprodukten des Methanols, des Methylacetats und des Dimethylethers.

Es ist allgemein bekannt, daß Methanol, Methylacetat und Dimethylether mit Kohlenmonoxid oder mit Kohlenmonoxid und Wasserstoff in Gegenwart von carbonylbildenden Metallen, hauptsächlich solchen der VIII. Gruppe des Periodensystems, auf vielfältige Weise zu verschiedenen Produkten reagieren können.

Diese Reaktionen werden üblicherweise unter dem Oberbegriff der Carbonylierung zusammengefaßt. Im einzelnen handelt es sich hierbei u. a. um die

- Carbonylierung (im engeren Sinne) von Methanol oder Dimethylether mit u. a. Co-, Ni- oder Rh-Katalysatoren zu Gemischen, die im wesentlichen aus Methanol, Essigsäure und Methylacetat bestehen, die
- Carbonylierung (im engeren Sinne) von Methylacetat mit u. a. Co-, Ni- oder Rh-Katalysatoren zu Gemischen, die im wesentlichen aus Methylacetat und Acetanhydrid bestehen, die
- Homologisierung von Methanol und Dimethylether mit $CO/H_2$ in Gegenwart von u. a. Co-, Ni- oder Rh-Katalysatoren, die je nach den Reaktionsbedingungen zu Gemischen führt, welche neben Methanol im wesentlichen Acetaldehyd, Ethanol, Methylacetat, Acetaldehyddimethylacetal und Wasser enthalten, und um die Homologisierung von Methylacetat mit $CO/H_2$ in Gegenwart von u. a. Co-, Ni- oder Rh-Katalysatoren, bei der man je nach den Reaktionsbedingungen Gemische erhält, die im wesentlichen aus Ethylidendiacetat, Vinylacetat, Methylacetat, Ethylacetat, Wasser und Methanol bestehen.

Für alle diese Verfahren sind eine Vielzahl von Varianten bekannt oder denkbar, so daß man entsprechend viele Reaktionsgemische unterschiedlicher qualitativer und quantitativer Zusammensetzung erhält. Den meisten Varianten dieser Carbonylierungsreaktionen ist jedoch gemeinsam, daß sie in Gegenwart von Jodiden vorgenommen werden, so daß die Reaktionsgemische stets merkliche Mengen an jodorganischen Verbindungen, wie vor allem Methyljodid, enthalten.

Diese jodorganischen Verbindungen lassen sich von den übrigen organischen Komponenten der Carbonylierungsgemische nur äußerst schwer abtrennen.

Die Fraktionierung, wenn wegen vielfältiger Azeotropbildung überhaupt möglich, erfordert einen unwirtschaftlich hohen Trennaufwand, und chemische Methoden wie Oxidation, Reduktion oder Alkalibehandlung kommen wegen der Empfindlichkeit mancher Carbonylierungsprodukte nicht in Betracht, ganz abgesehen davon, daß sie verfahrenstechnisch umständlich sind und meistens nur ein schwieriges Trennproblem durch im Prinzip weniger schwierige ersetzen.

Beispielsweise entfernt man das Methyljodid von Carbonylierungsprodukten des Methylacetats nach der Lehre der DE-OS 2 940 751 durch Umsetzung mit Alkalimetallacetaten, wobei man Alkalimetalljodide erhält. Da diese Umsetzung jedoch nur bei erhöhter Temperatur stattfindet, muß man sie unter Druck vornehmen, und danach müssen die Jodsalze abgetrennt werden. Diese Maßnahmen sowie auch die anschließende Rückführung der Jodide in den Verfahrenskreislauf sind jedoch verfahrenstechnisch aufwendig und unbefriedigend und lassen sich vor allem nicht harmonisch in einen kontinuierlichen Ablauf einfügen. Ist schließlich Wasser als Komponente des Reaktionsgemisches zugegen, gelingt diese Methode der Jodentfernung praktisch überhaupt nicht mehr.

Der Erfindung lag daher die Aufgabe zugrunde, Methyljodid sowie sonstige organische Jod-Verbindungen aus Reaktionsgemischen der Carbonylierung von Methanol, Methylacetat und Dimethylether auf einfachere und wirtschaftlichere Weise zu entfernen als bisher.

Demgemäß wurde gefunden, daß man Methyljodid sowie sonstige organische Jod-Verbindungen auf elegante Weise von acetaldehydfreien Reaktionsgemischen der Carbonylierung von Methanol, Methylacetat und Dimethylether abtrennen kann, wenn man die Jod-Verbindungen durch azeotrope Destillation mit einem Kohlenwasserstoff, der unter Normaldruck bei 25–55° C siedet, entfernt.

Überraschenderweise ist das erfindungsgemäße Verfahren nicht nur auf bestimmte Carbonylierungsprodukte des Methanols, Methylacetats und Dimethylethers anwendbar, sondern auch auf beliebige Gemische, die bei diesen Reaktionen anfallen. Die weitere Aufarbeitung solcher Gemische ist somit nicht mehr mit dem Problem der Jodentfernung belastet und kann daher auf beliebige Weise vorgenommen werden.

Enthalten die Gemische Acetaldehyd, so trennt man diesen vorher zusammen mit einem Teil der Jodverbindungen ab und unterwirft ihn zweckmäßigerweise dem Reinigungsverfahren durch Azeotropdestillation mit den im vorliegenden Fall verwendeten Kohlenwasserstoffen. Diese Kohlenwasserstoffe bilden mit Acetaldehyd ebenfalls Azeotrope oder azeotrop-ähnliche Gemische.

Aus diesen wird der Acetaldehyd mit Wasser ausgewaschen, wonach die Kohlenwasserstoffe wieder in die Azeotropdestillation zurückgeführt werden.

2

**0 053 249**

Für die erfindungsgemäße Azeotropdestillation der acetaldehydfreien Carbonylierungsprodukte bzw. von deren Gemischen verwendet man pro Gramm der Jodverbindungen — hauptsächlich handelt es sich um Methyljodid — etwa 10–200 g des Kohlenwasserstoffs, wobei Isopentan (Methylbutan) an erster Stelle zu nennen ist. Weitere geeignete Kohlenwasserstoffe sind n-Pentan, Cyclopentan und 2,2-Dimethylbutan.

Die Trennung der Gemische aus den Jod-Verbindungen und den Kohlenwasserstoffen von den Carbonylierungsprodukten ist unproblematisch und erfordert im allgemeinen lediglich Kolonnen beliebiger Bauart mit etwa 20–40 theoretischen Böden. Die Azeotrope sieden bei 25–50°C, also niedriger als Methylacetat, die Komponente mit dem tiefsten Siedepunkt im flüssigen Reaktionsaustrag. Führt man die jodhaltigen Azeotrope in die Synthesestufe zurück, brauchen sie nicht kondensiert zu werden, so daß sich im allgemeinen das Arbeiten unter Normaldruck empfiehlt. Will man sie dagegen kondensieren, so ist es zweckmäßig, bei etwa 1,5–3 bar zu arbeiten, um dadurch die Kühlung mit Wasser normaler Temperatur zu ermöglichen.

Aus den jodhaltigen Gemischen, in denen die Konzentration der Jod-Verbindungen etwa 0,5–2 Gew.-% und höher beträgt, kann das Jod auf beliebige chemische Weise, z. B. durch Behandlung mit Alkalimetallen oder Alkalilaugen und anschließende Abtrennung der Jodide von den inerten Kohlenwasserstoffen, entfernt werden. Besonders empfiehlt es sich jedoch, die jodhaltigen Kohlenwasserstoffe direkt in die Stufe der Carbonylierungsreaktion zurückzuführen. Auf diese Weise entsteht ein geschlossener Kreislauf sowohl des Jods als auch der Kohlenwasserstoffe.

Das erfindungsgemäße Verfahren ist prinzipiell unabhängig davon, unter welchen Bedingungen und mit welcher Zielsetzung die Carbonylierung vorgenommen wurde, sofern überhaupt Jod-Verbindungen als Aktivatoren im System der Carbonylierungskatalysatoren mitverwendet wurden, wie es für die Mehrzahl der technisch ausgeübten Synthesen dieser Art zutrifft. Es erübrigt sich daher, die verschiedenartigen Reaktionsbedingungen für die eingangs erwähnten Varianten der Carbonylierungsreaktionen im einzelnen aufzuführen.

Das erfindungsgemäße Verfahren ermöglicht es auf denkbar einfache Weise, den Jodgehalt der Carbonylierungsprodukte bzw. von deren Gemischen von etwa 1000–10 000 ppm auf 0,1–2 ppm zu senken.

## Beispiel

Verschiedene Methyljodid enthaltende Modellmischungen, deren Komponenten nach Art und Menge etwa Carboxylierungsgemischen entsprechen, wurden mit Isopentan versetzt und sodann in einer Füllkörperkolonne mit etwa 28 theoretischen Böden bei dem Rücklaufverhältnis r kontinuierlich in eine Isopentan-Kopffraktion und eine Sumpffraktion, die bis auf den Methyljodidgehalt der ursprünglichen Mischung entsprach, zerlegt.

Der Methyljodidgehalt in den beiden Fraktionen wurde jeweils gaschromatographisch ermittelt.

Die Tabelle zeigt die Bedingungen und Ergebnisse der einzelnen Versuche.

Tabelle

| Versuch Nr. | Ausgangsprodukt bzw. -gemisch (Zulauf) | Isopentan | r | Methyljodid-Gehalt [ppm] | |
|---|---|---|---|---|---|
| | g/h | g/h | | Zulauf | Sumpffraktion |
| 1 | 180 Essigsäure | 55 | 0,5 | 4000 | 1—2 |
| 2 | 100 Essigsäure<br>100 Methylacetat | 57 | ·1,0 | 5000 | 0,1 |
| 3 | 95 Essigsäureanhydrid<br>95 Methylacetat | 60 | 1,0 | 5000 | 0,1—0,8 |
| 4 | 57 Essigsäureanhydrid<br>57 Methylacetat<br>57 Ethylidendiacetat | 55 | 1,0 | 7000 | 0,1 |
| 5 | 50 Essigsäureanhydrid<br>50 Ethylidendiacetat<br>50 Vinylacetat<br>50 Essigsäure | 37 | 1,0 | 1000 | 1 |
| 6 | 67 Essigsäureanhydrid<br>67 Ethylidendiacetat<br>67 Methylacetat | 44 | 1,0 | 2000 | 1 |

**Patentansprüche**

1. Verfahren zur Abtrennung organischer Jod-Verbindungen von acetaldehydfreien Carboxylierungsprodukten des Methanols, Methylacetats und Dimethylethers, dadurch gekennzeichnet, daß man die Jod-Verbindungen durch azeotrope Destillation mit einem Kohlenwasserstoff, der unter Normaldruck bei 25 – 55° C siedet, entfernt.

2. Verfahren nach Anspruch 1, dadurch gekennzeichnet, daß man als Kohlenwasserstoff Isopentan (2-Methylbutan) verwendet.

**Claims**

1. A process for separating off organic iodine compounds from acetaldehyde-free carboxylation products of methanol, methyl acetate and dimethyl ether, wherein the iodine compounds are removed by azeotropic distillation with a hydrocarbon having a boiling point, under atmospheric pressure, of 25 – 50° C.

2. A process as claimed in claim 1, wherein isopentane (2-Methylbutane) is used as the hydrocarbon.

**Revendications**

1. Procédé de séparation de composés organiques iodés des produits de carbonylation de l'alcool méthylique, de l'acétate de méthyle et de l'éther diméthylique, exempts d'aldéhyde acétique, caractérisé en ce que l'on extrait les composés iodés par distillation azéotropique avec un hydrocarbure d'un point d'ébullition de 25 à 55° C à la pression normale.

2. Procédé suivant la revendication 1, caractérisé en ce que l'hydrocarbure utilisé est l'isopentane (méthyl-2 butane).